# EUROPEAN PATENT APPLICATION

(11) **EP 2 551 339 A1**
(43) Date of publication of application: **30.01.2013**
(21) Application number: 11759369.9
(22) Date of filing: 22.03.2011
(51) Int. Cl.: C12M 1/26, C12M 1/34, G01N 1/22

(54) **COLLECTOR FOR SUBSTANCES TO BE DETECTED AND METHOD FOR USING SAME**

(30) Priority: 23.03.2010 JP 2010065533
(71) Applicant: Hitachi Plant Technologies, Ltd., Tokyo 170-8466 (JP)
(72) Inventor: MIYASHITA Noe, Tokyo 170-8466 (JP); TAZAKI Aya, Tokyo 170-8466 (JP); KAMITANI Matsuo, Tokyo 170-8466 (JP); OKANOJO Masahiro, Tokyo 100-8220 (JP); NODA Hideyuki, Tokyo 100-8220 (JP)
(74) Representative: Beetz & Partner
(86) International application number: PCT/JP2011/056763
(87) International publication number: WO 2011/118564

(57) **Abstract**

Provided is a device for capturing object, wherein a carrier which captures substances to be detected and is made to be dividable into plural portions, is placed with the plural dividable portions arranged in sections. The carrier includes a count analysis carrier and an identification analysis carrier respectively arranged in sections in a first dish half and a second dish half which are obtained by dividing a capturing dish.

## Description

### TECHNICAL FIELD

The present invention relates to a device for capturing object which captures objects such as microorganisms and chemical substances, and a method for using the device for capturing object.

### BACKGROUND ART

Conventionally, a technique of capturing objects, such as airborne microorganisms and chemical substances, by sucking air through a filter and separating the objects by the filter has been well-known. A well-known device for capturing air-borne microorganisms has a carrier, which undergoes a phase transition from gel to sol at a temperature raised from room temperature, on a capturing dish (refer to, for example, Patent Document 1) . Such a device for capturing object is attached to an impactor-type air sampler. When air sucked by the air sampler collides with the carrier, microorganisms carried by the air flow are captured by the carrier in a gel phase. The carrier solates by raising the temperature, and thereby the captured microorganisms with the carrier in a sol phase are obtained from the capturing dish. The obtained microorganisms are counted according to a predetermined counting method.

A well-known method for counting microorganisms is the ATP method, which quantifies adenosine triphosphates (ATPs) extracted from microorganisms and thereby indirectly counts the microorganisms (refer to, for example, Patent Document 2) . The ATP method extracts ATPs contained in the microorganisms by contacting the captured microorganisms with an ATP extracting reagent, and counts the microorganisms based on the intensity of luminescence measured when the extracted ATPs reacts with a luminescence reagent.

It takes several days to obtain a counting result by, for example, a method for counting captured microorganisms based on the number of microorganism colonies cultured in a plate medium. On the other hand, the ATP method requires about one to several hours from when microorganisms are captured until the microorganisms are counted. Thus, the ATP method dramatically reduces the required time.
However, the ATP method counts microorganisms based on weak luminescence intensity. Substances that act as disturbance factors may be contained in a sample to be counted. Those substances thus need to be minimized.

### RELATED ART DOCUMENTS

### PATENT DOCUMENTS

Patent Document 1: Japanese Patent Application Laid-Open No. 2009-131186
Patent Document 2: Japanese Patent Application Laid-Open No. 11-137293

### SUMMARY OF THE INVENTION

### PROBLEM TO BE SOLVED BY THE INVENTION

A conventional device for capturing object (refer to, for example, Patent Document 1) has a carrier exposed on a capturing dish. Thus, for example, microorganisms other than those to be tested, or other substances that act as disturbance factors may attach onto the exposed carrier during the time after microorganisms are captured onto the carrier in an air sampler and before the microorganisms are counted. Particularly, when a test site where microorganisms are captured is far from a site where the microorganisms are counted, a possibility of contamination of the carrier may be further increased.
In other words, when the conventional device for capturing object (refer to, for example, Patent Document 1) is used, the carrier may be contaminated during the time after the carrier is removed from the air sampler and before the microorganisms are counted, and thereby the microorganisms captured at the test site may not be accurately counted.

In light of the described above, prior to this application, the present applicants already filed a patent application related to a device for capturing object which is configured to operate to capture objects in a state where a carrier of a capturing dish is directed upward and to detect the objects in a state where the carrier is directed downward (Japanese Patent Application No. 2009-295655).
According to the device for capturing object, the carrier after subjected to operations of capturing objects is disposed in the state where the carrier is directed downward. This makes the capturing dish itself a cover of the carrier, which can prevent substances as disturbance factors from being mixed into the carrier.

On the other hand, in the device for capturing object as described above, there is a desire that a quantitative analysis and a qualitative analysis of objects captured by a carrier be performed in parallel. For example, if the captured objects are microorganisms, there is a desire that a counting of the microorganisms and an identification of the microorganisms be performed in parallel.
Further, a carrier (sample) of detected substances subjected to a quantitative analysis is required to be captured at the same time and at the same place as that of a carrier (sample) subjected to a qualitative analysis. One of the solutions may be that a single carrier is used for capturing objects and is then cut into, for example, one portion for use in a quantitative analysis and another for use in a qualitative analysis.
If the carrier is used after cutting, however, there is a possibility that, when the carrier is cut into portions or the cut portion for the quantitative analysis is weighed, substances that act as disturbance factors are contained in the carrier, thus making it difficult to perform an accurate analysis.

The present invention has been made in an attempt to provide: a device for capturing object which can further accurately analyze a carrier with which substances to be detected are captured, when the substances to be detected are subjected to a plurality of analyses, for example, a quantitative analysis and a qualitative analysis; and a method for using the device for capturing object.

### MEANS FOR SOLVING THE PROBLEM

To solve the above problems, the present invention provides a device for capturing object in which a carrier made to be dividable into plural portions is placed with the plural dividable portions arranged in sections.

To solve the above problems, the present invention also provides a method for using the device for capturing object in which a carrier made to be dividable into plural portions is placed with the plural dividable portions arranged in sections.

### EFFECTS OF THE INVENTION

The present invention can provide: a device for capturing object which can further accurately analyze a carrier with which substances to be detected are captured, when the substances to be detected are subjected to a plurality of analyses, for example, a quantitative analysis and a qualitative analysis; and a method for using the device for capturing object.

### BRIEF DESCRIPTION OF THE DRAWINGS

FIG. 1 is a diagram showing a structure of a microorganism counting apparatus having a device for capturing object mounted therein according to a first embodiment of the present invention.
FIG. 2 is a perspective view showing the vicinity of a mounting unit for the device for capturing object in the microorganism counting apparatus of FIG. 1 according to the first embodiment.
FIG. 3 is a cross-sectional view showing the device for capturing object mounted on the mounting unit in the microorganism counting apparatus of FIG. 1 according to the first embodiment.
FIG. 4 is a flowchart of operations of the microorganism counting apparatus based on instructions of a control unit according to the first embodiment.
FIG. 5 is a perspective view showing the device for capturing object according to the first embodiment.
FIG. 6A is an exploded perspective view showing the device for capturing object of FIG. 5 viewed from obliquely above according to the first embodiment. FIG. 6B is an exploded perspective view showing the device for capturing object of FIG. 5 viewed from obliquely below according to the first embodiment.
FIG. 7 is a cross-sectional view along a line VII-VII in FIG. 5 according to the first embodiment.
FIG. 8 is a perspective view showing a method for capturing microorganisms using the device for capturing object according to the first embodiment.
FIG. 9 is a perspective view showing how a carrier is divided in the device for capturing object according to the first embodiment.
FIG. 10A1 to FIG. 10A4 are each a cross-sectional view of the device for capturing object, showing a method for using the device for capturing object in the microorganism counting apparatus according to the first embodiment. FIG. 10B1 to FIG. 10B4 are each an enlarged schematic diagram showing the vicinity of a filter in the cases of FIG. 10A1 to FIG. 10A4, respectively, according to the first embodiment.
FIG. 11 is an exploded plan view showing a structure of a capturing dish according to a variation.
FIG. 12A is an exploded perspective view showing a device for capturing object viewed from obliquely above according to a second first embodiment. FIG. 12B is an exploded perspective view showing the device for capturing object viewed from obliquely below according to the second embodiment.
FIG. 13 is a cross-sectional view along a line VII-VII in FIG. 5 according to the second embodiment.
FIG. 14 is a perspective view showing how a carrier is divided in the device for capturing object according to the second embodiment.

### EMBODIMENTS FOR CARRYING OUT THE INVENTION

A device for capturing object according to an embodiment of the present invention will be described in detail with reference to the drawings as appropriate. The embodiment will be described using a device for capturing object for capturing air-borne microorganisms (for example, microbes and fungi) as an example. However, the objects captured by the device for capturing object of the present invention may be microscopic particles of metal or of chemical substances. The objects are not limited to solid objects, and may be mist.

First will be described an overall structure of a microorganism counting apparatus equipped with the device for capturing object according to the embodiment, and a method for counting microorganisms by the microorganism counting apparatus. Second will be described the device for capturing object according to the first embodiment and a method for using the device for capturing object.

### <Overall Structure of Microorganism Counting Apparatus>

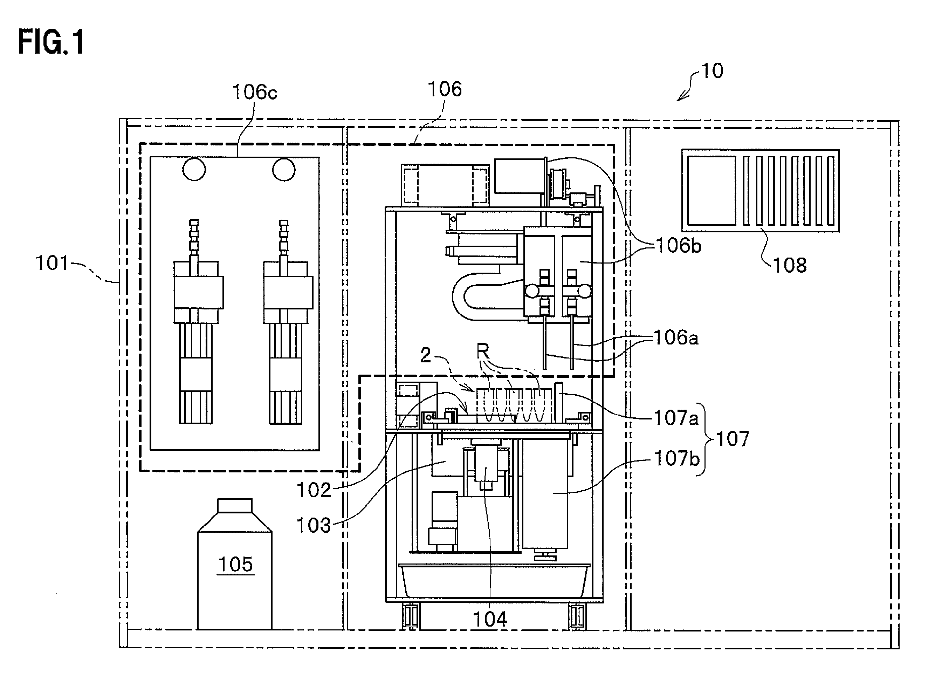

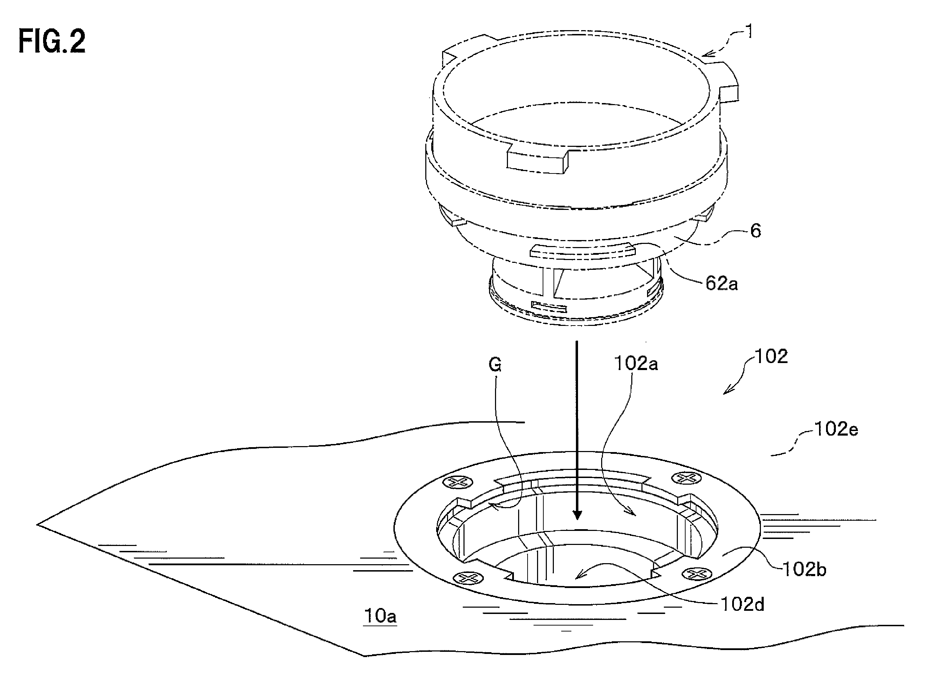

As shown in FIG. 1, a microorganism counting apparatus 10 is an apparatus for counting microorganisms contained in a sample according to the ATP method. The microorganism counting apparatus 10 includes a mounting unit 102 mounting a device for capturing object 1 (refer to FIG. 2) having the sample therein, a liquid tank 105, a hot-water supplying unit 103, a suction unit 104, a reagent cartridge 2 having multiple reagents R, a dispensing unit 106, a luminescence-intensity measurement unit 107, and a control unit 108, which are housed in a cabinet 101.
For simplicity, FIG. 1 shows the cabinet 101 and the reagent cartridge 2 in a dashed-two-dotted line, and omits the device for capturing object 1.

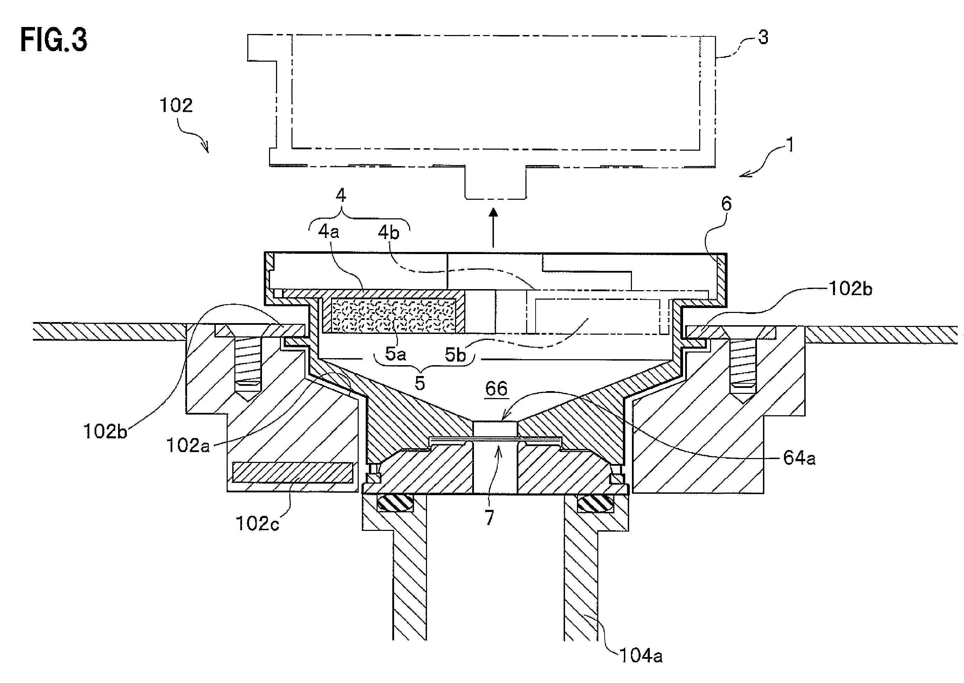

As shown in FIG. 2, the mounting unit 102 has a recessed portion 102a receiving the device for capturing object 1 (a housing 6) . The mounting unit 102 also includes an engaging ring 102b. As described below, a heater 102c (refer to FIG. 3) is embedded in an aluminum member surrounding the recessed portion 102a, that is, forming the recessed portion 102a. To form the recessed portion 102a, other highly heat conductive material may be used instead of the aluminum member.

The engaging ring 102b is attached on the periphery of the opening of the recessed portion 102a. As described in detail below, the housing 6 is mounted on the mounting unit 102 by engaging the engaging ring 102b with first engaging claws 62a provided on the housing 6 of the device for capturing object 1. The engaging ring 102b has cutout portions 102d in such a planar shape as to receive respective first engaging claws 62a of the housing 6. Between the engaging ring 102b and an apparatus body 10a having the recessed portion 102a formed therein, a gap G is formed to have a height large enough for receiving the first engaging claws 62a.
In other words, when the housing 6 is fitted into the recessed portion 102a, the first engaging claws 62a are inserted into the recessed portion 102a through respective cutout portions 102d, and the housing 6 is rotated to slide the first engaging claws 62a into the gap G. Thereby, the housing 6 is engaged with the engaging ring 102b.

As shown in FIG. 3, when the device for capturing object 1 is placed in the recessed portion 102a, a cover 3 of the device for capturing object is removed. Herein, the capturing dish 4 includes a first dish half 4a and a second dish half 4b and the first dish half 4a has a count analysis carrier 5a. The count analysis carrier 5a in the first dish half 4a in the housing 6 is disposed in such a way as to expose in an internal space 66 of the housing 6, which will be described later in detail.
Note that in a state shown in FIG. 3, the second dish half 4b and an identification analysis carrier 5b which constitutes carrier 5 are not present in the housing 6 and are thus shown in a dashed-two-dotted line.

In FIG. 3, a discharge opening 64a of the housing 6, a filter 7 provided on the outside of the discharge opening 64a, and a suction head 104a of the suction unit 104 (refer to FIG. 1) connected with the housing 6 are shown. The internal space 66 in the housing 6 may also be referred to as "a space".

The heater 102c may be any means that is capable of heating the recessed portion 102a (the aluminum member), which surrounds the housing 6 of the device for capturing object 1 mounted on the mounting unit 102, to a predetermined temperature. Specifically, the heater 102c is preferable to be, for example, a cartridge heater.

The liquid tank 105 shown in FIG. 1 is adapted to store liquid such as sterile distilled water. This liquid is poured into the housing 6 (refer to FIG. 3) so as to, for example, improve a filtration rate of the count analysis carrier 5a (refer to FIG. 3) of the device for capturing object 1, or for washing, as described below. The liquid is also poured into a piping system connected to a syringe pump 106c of the dispensing unit 106 as described below. The liquid tank 105 may store buffer solution.

The hot-water supplying unit 103 shown in FIG. 1 heats and supplies, for example, the sterile distilled water supplied from the liquid tank 105. More specifically, the hot-water supplying unit 103 injects hot water into the internal space 66 (refer to FIG. 3) in the housing 6. For example, the hot-water supplying unit 103 is, though not shown, a unit for discharging hot water heated by a cartridge heater with a peristaltic pump through a nozzle formed with a flexible tube or the like. The nozzle having a means for moving in horizontal and vertical directions is inserted into the internal space 66 (refer to FIG. 3) of the housing 6 as necessary.

The suction unit 104 shown in FIG. 1 sucks, for example, the hot water and the reagents R described below, which are injected into the internal space 66 (refer to FIG. 3) in the housing 6, to discharge them through the filter 7 (refer to FIG. 3) . For example, the suction unit 104 includes the suction head 104a (refer to FIG. 3) described above, a suction pump, not shown, connected with the suction head 104a through predetermined piping, and a waste tank.
The suction unit 104 according to this embodiment further includes a lifting apparatus (not shown) lifting and lowering the suction head 104a (refer to FIG. 3) to enable the suction head 104a to be engaged with and disengaged from the housing 6 (refer to FIG. 3) mounted on the mounting unit 102.

In the reagent cartridge 2 shown in FIG. 1, multiple reagents R necessary to the ATP method are arranged in a block. The reagent cartridge 2 is disposed at a predetermined position in the vicinity of the mounting unit 102. In the reagent cartridge 2, each reagent R is disposed at a predetermined position, and a dispensing nozzle 106a of the dispensing unit 106 described below dispenses the reagents R in a predetermined order into the housing 6 of the device for capturing object 1. In other words, the location (coordinates) of each reagent R is stored in the control unit 108 controlling the dispensing unit 106, as described below.

Examples of the reagents R necessary to the ATP method includes an ATP eliminating reagent for eliminating ATPs that are present outside cells of captured microorganisms, an ATP extracting reagent for extracting ATPs contained in the microorganisms.
Examples of the ATP eliminating reagent include an ATP-degrading enzyme.
Examples of the ATP extracting reagent include a benzalkonium chloride, a trichloroacetic acid, and a Tris buffer solution.
The examples of the reagents R may include a correction reagent for the luminescence-intensity measurement unit 107, and sterile pure water. Meanwhile, an ATP luminescence reagent which makes ATPs extracted from microorganisms luminescent is provided in a luminescence-test tube 107a (refer to FIG. 1) in which the ATP luminescence reagent at a predetermined concentration is previously filled, which is to be described hereinafter. Thus, the ATP luminescence reagent is no arranged in the reagent cartridge 2.
Examples of the ATP luminescence reagent include a luciferase/luciferin reagent.

The dispensing unit 106 shown in FIG. 1 dispenses the reagents R described above into the housing 6 of the device for capturing object 1. The dispensing unit 106 also dispenses the reagents R or ATPs extracted from microorganisms by an ATP extract solution (the ATP extract solution containing the ATPs) in the housing 6 (refer to FIG. 3) as described below, into the luminescence-test tube 107a disposed on the luminescence-intensity measurement unit 107.

The dispensing unit 106 may include the dispensing nozzle 106a formed with a thin tube, an actuator 106b moving the dispensing nozzle 106a in the xyz axis directions, a syringe pump 106c connected with the dispensing nozzle 106a through predetermined flexible piping, the piping, not shown, supplying sterile distilled water or the like from the liquid tank 105 through the syringe pump 106c to the dispensing nozzle 106a.

The luminescence-intensity measurement unit 107 shown in FIG. 1 may be a unit that includes: the luminescence-test tube 107a which contains an ATP luminescence reagent by receiving the ATP extract solution containing ATPs dispensed from the housing 6 (refer to FIG. 3); and a luminescence detecting unit body 107b which has a photomultiplier or the like for detecting luminescence intensity of the ATPs.

The control unit 108 shown in FIG. 1 has overall control over the microorganism counting apparatus 10. The control unit 108 also controls the hot-water supplying unit 103, the suction unit 104, the dispensing unit 106, and the luminescence-intensity measurement unit 107 according to a procedure to be described below, after the device for capturing object 1 (refer to FIG. 3) is mounted on the mounting unit 102. This control unit 108 includes a CPU, a ROM, a RAM, various interfaces, and circuitry.

### <Operations of Microorganism Counting Apparatus and Method for Counting Microorganisms>

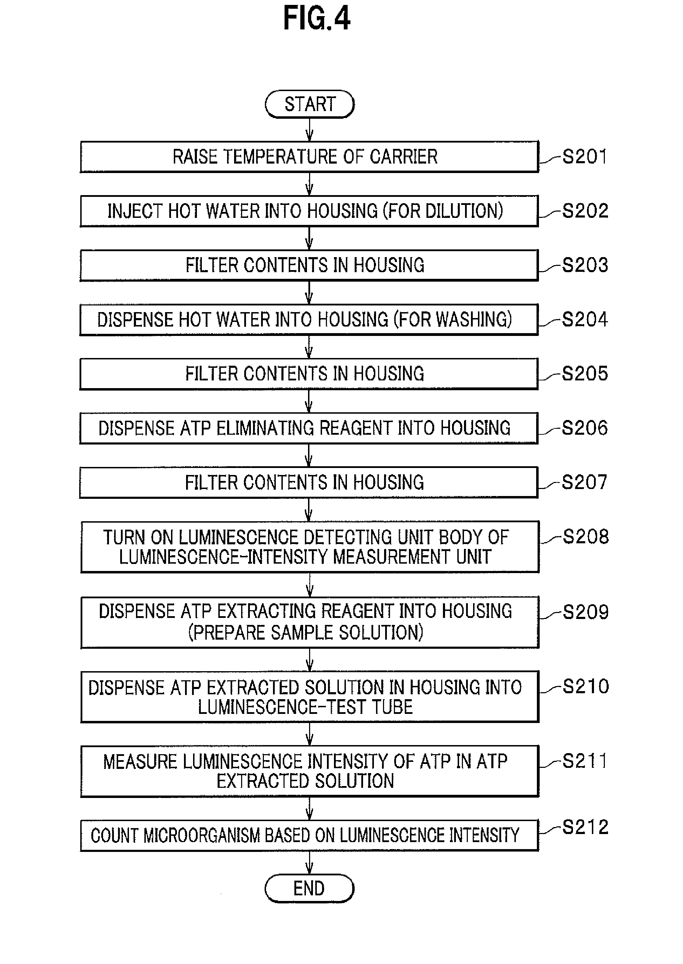

A procedure of execution by the control unit 108 will be described next. In the description, operations of the microorganism counting apparatus 10 and a method for counting microorganisms will be described with reference mainly to FIG. 4. FIG. 4 is a flowchart showing the procedure in which the microorganism counting apparatus operates based on instructions of the control unit.

In the microorganism counting apparatus 10 shown in FIG. 1, the control unit 108 starts execution of the following procedure when a start switch, not shown, is turned on after the device for capturing object 1 (refer to FIG. 3) is mounted on the mounting unit 102.

As shown in FIG. 4, the control unit 108 sends an instruction to, for example, a predetermined inverter to apply power to the heater 102c (refer to FIG. 3) to generate heat. On the request of the control unit 108, the temperature of the count analysis carrier 5a (refer to FIG. 3) of the device for capturing object 1 is raised with the heater 102c as shown in FIG. 4 (Step S201). Thereby, the count analysis carrier 5a solates and falls down off the first dish half 4a (refer to FIG. 3) onto the inside bottom of the housing 6 (refer to FIG. 3).

The control unit 108 sends an instruction to the hot-water supplying unit 103 (refer to FIG. 1) to inject hot water into the housing 6 (refer to FIG. 3) (Step S202) . Thereby, the count analysis carrier 5a (refer to FIG. 3) further solates and is diluted with the hot water.

The control unit 108 sends an instruction to the suction unit 104 (refer to FIG. 1) to engage the suction head 104a (refer to FIG. 3) with the housing 6 (refer to FIG. 3), and then suck and filter the contents in the housing 6 (refer to FIG. 3) (Step S203). Thereby, the microorganisms captured in the count analysis carrier 5a are separated and held by the filter 7 (refer to FIG. 3), and the diluted count analysis carrier 5a is filtered and discharged out of the housing 6.

The control unit 108 again sends an instruction to the hot-water supplying unit 103 to dispense hot water into the housing 6 (refer to FIG. 3) (Step S204). Then, the hot water in the housing 6 is filtered again (Step S205). Thereby, the filtered hot water removes remaining diluted carrier 5 from the inside of the housing 6, and accordingly the recovery rate of microorganisms at the filter 7 is improved.

The control unit 108 sends an instruction to the dispensing unit 106 (refer to FIG. 1) to dispense the ATP eliminating reagent in the reagent cartridge 2 into the housing 6 (refer to FIG. 3) (Step S206) . As a result, the ATPs that are present outside the cells of the microorganisms on the filter 7 are eliminated.

The control unit 108 sends an instruction to the suction unit 104 (refer to FIG. 1) to suck the contents of the housing 6 (refer to FIG. 3) and filter the sucked contents (Step S207). Thereby, the microorganisms are separated and held by the filter 7 (refer to FIG. 3), and the ATP eliminating reagent is filtered and discharged out of the housing 6.

The control unit 108 sends an instruction to the luminescence-intensity measurement unit 107 (refer to FIG. 1) to turn on the luminescence detecting unit body 107b (refer to FIG. 1) (Step S208). Thereby, a background measurement of the luminescence-test tube 107a which has been previously disposed above the luminescence-intensity measurement unit 107 and contains the ATP luminescence reagent is performed.

The control unit 108 sends an instruction to the dispensing unit 106 (refer to FIG. 1) to dispense the ATP luminescence reagent in the reagent cartridge 2 into the housing 6 (refer to FIG. 3) (Step S208). Thereby, ATPs are extracted from the microorganisms held by the filter 7, and a sample solution is prepared on the filter 7.

The control unit 108 sends an instruction to the dispensing unit 106 (refer to FIG. 1) to dispense the ATP extract solution containing ATPs in the housing 6 (refer to FIG. 3), into the luminescence-test tube 107a at which the background measurement is performed (Step S210). Thereby, the ATPs in the ATP extract solution react with the ATP luminescence reagent and produces luminescence in the luminescence-test tube 107a.

The luminescence detecting unit body 107b (refer to FIG. 1) detects the ATP luminescence and outputs signals. The control unit 108 digitizes the outputted signals and measures luminescence intensity based on the single-photon counting method (Step S211) . Then, the control unit 108 calculates the ATP amount (amol) in the ATP extract solution dispensed into the luminescence-test tube 107a based on a prestored calibration curve indicating the relation between the ATP amount (amol) and the luminescence intensity (CPS) . The control unit 108 then counts the microorganisms using an ATP value which is converted into the equivalent number of the microorganisms in the carrier 5. The ATP value is calculated based on the ATP amount (amol) and the amount of the ATP extract solution of the sample solution prepared in Step S209 (Step S212).

### <Device for Capturing Object>

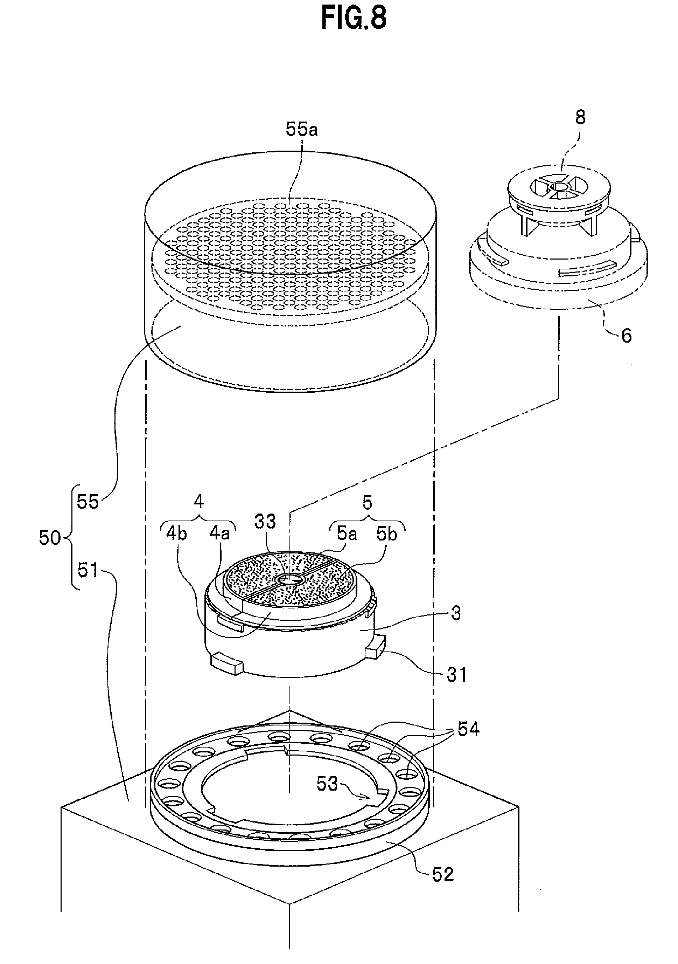

The device for capturing object 1 according to the first embodiment of the present invention (refer to FIG. 2) has a carrier for capturing microorganisms as air-borne objects. The carrier made to be dividable into plural portions is placed with the plural dividable portions arranged in sections.
The device for capturing object 1 is placed in an impactor-type air sampler 50 (refer to FIG. 8) to capture microorganisms which are air-borne objects. The device for capturing object 1 is mounted in the microorganism counting apparatus 10 described above to count the captured microorganism. The device for capturing object 1 is turned upside down and used when microorganisms are captured, as described in detail below.

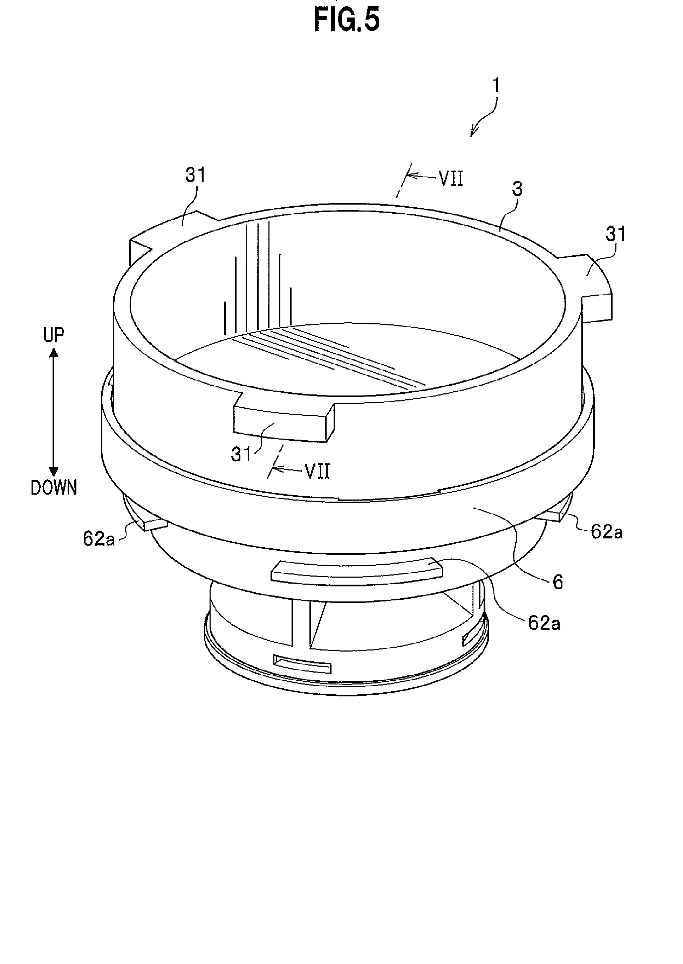

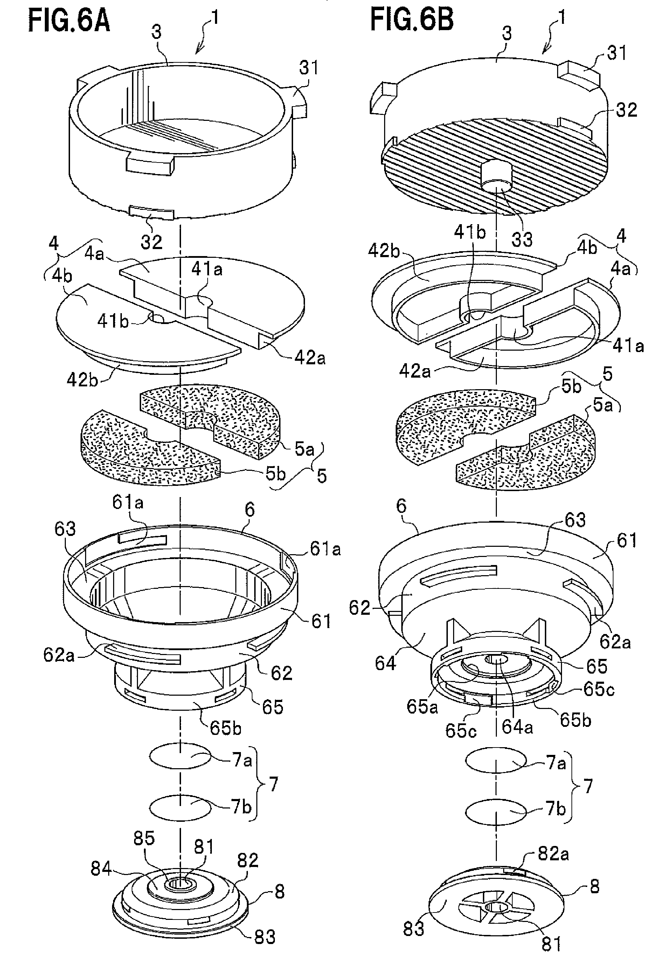

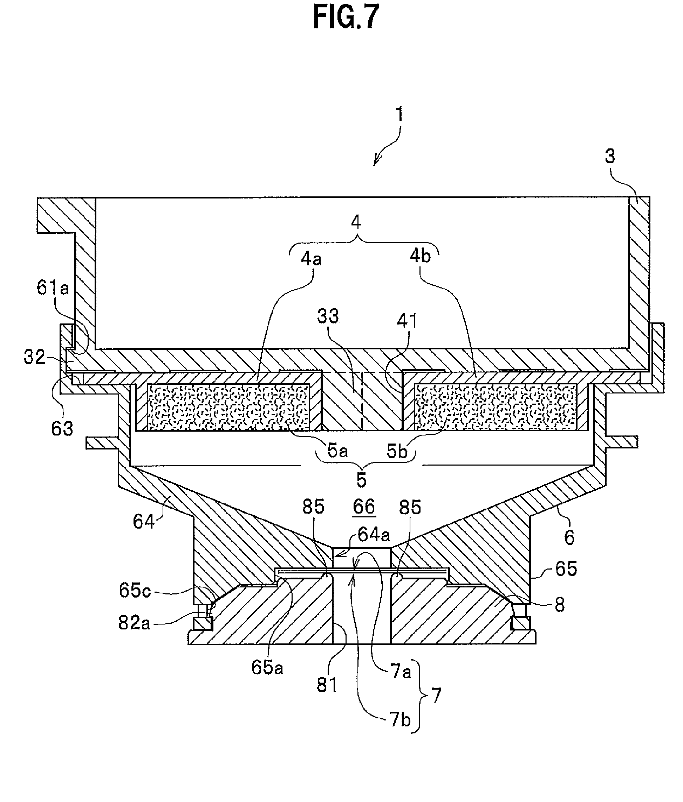

FIG. 5 which is referred to next is a perspective view showing the device for capturing object according to the first embodiment of the present invention. FIG. 6A is an exploded perspective view showing the device for capturing object of FIG. 5 viewed from obliquely above. FIG. 6B is an exploded perspective view showing the device for capturing object of FIG. 5 viewed from obliquely below. FIG. 7 is a cross-sectional view along a line VII-VII in FIG. 5. The up and down direction of the device for capturing object 1 in the following description is the same as that shown in FIG. 5.

As shown in FIG. 5, an upper portion of the device for capturing object 1 according to the first embodiment is formed in a substantially cylindrical shape. A lower portion of the device for capturing object 1 is formed in an inverted conical shape such that the diameter of the horizontal cross section of the device for capturing object 1 becomes smaller downward as the horizontal cross section lowers. As described in detail below, the upper portion of the device for capturing object 1 is engaged with the air sampler 50 (refer to FIG. 8) and is used for capturing microorganisms. The lower portion of the device for capturing object 1 is engaged with the microorganism counting apparatus 10 (refer to FIG. 8) and is used for counting the captured microorganisms.

In FIG. 5, reference numeral 3 indicates the cover; reference numeral 6 indicates the housing; reference numeral 31 indicates second engaging claws 31 engaging with the air sampler 50 (refer to FIG. 8) described below; and reference numeral 62a indicates the first engaging claws engaging with the microorganism counting apparatus 10.

As shown in FIGS. 6A and 6B, the device for capturing object 1 includes the cover 3, the capturing dish 4, the carrier 5, the housing 6, the filter 7, and a filter-securing ring 8, which are disposed in this order from upward to downward and are fitted with each other.

As shown in FIGS. 6A and 6B, the cover 3 is attached to close an upper opening of the housing 6 described below and has a cylindrical shape with a bottom and an opening facing upward. On an upper circumferential edge of the outer cylindrical surface of the cover 3, the second engaging claws 31 described above are formed to protrude radially outward and are disposed in a constant spacing with each other on the circumferential surface of the cover 3. In this embodiment, the number of the second engaging claws 31 is three in accordance with the number of cutout portions 53 (refer to FIG. 8) described below of the air sampler 50.

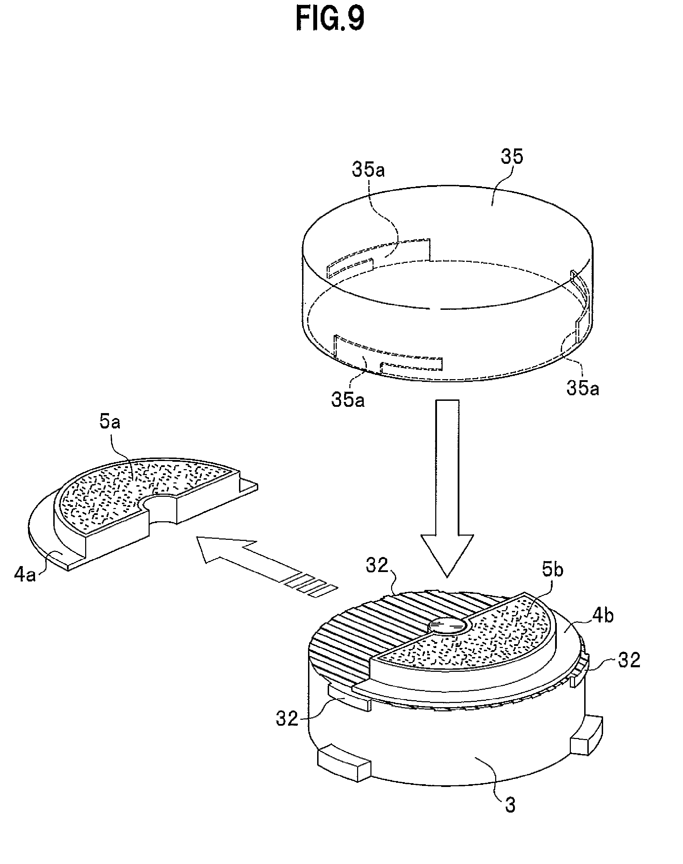

As shown in FIGS. 6A and 6B, on a lower circumferential edge of the outer cylindrical surface of the cover 3, three third engaging claws 32 are formed to protrude radially outward and are disposed in a constant spacing with each other on the circumferential surface of the cover 3. The third engaging claws 32 are fitted in respective first L-shaped grooves 61a described below of the housing 6 to detachably engage the cover 3 with the housing 6. The third engaging claws 32 are also fitted in respective third L-shaped grooves 35a (refer to FIG. 9) described below of the first cover body 35 to detachably engage the cover 3 with the first cover body 35.

As shown in FIG. 6B, an outer bottom surface of the cover 3 forms an uneven surface constituted by multiple straight ridges protruding downward and straight grooves disposed alternately and in parallel with each other. When the outer (lower) bottom surface of the cover 3 is brought in contact with an upper surface of the capturing dish 4 (the first dish half 4a and the second dish half 4b) as described below, the uneven outer bottom surface reduces the area of contact with the capturing dish 4. When the cover 3 is removed from the housing 6, the uneven surface facilitates easy detachment of the cover 3 from the capturing dish 4 which is in turn left in the housing 6. As described below, after microorganisms are captured using the air sampler 50 (refer to FIG. 8), when the device for capturing object 1 is carried to a microorganism counting facility (for example, a facility having the microorganism counting apparatus 10 (refer to FIG. 1)) at low temperature as necessary, condensation may rarely occur between the cover 3 and the capturing dish 4. Even in this case, the uneven surface facilitates easy detachment of the cover 3 from the capturing dish 4. This uneven surface is not limited to the above straight ridges and straight grooves, and may be formed with multiple protrusions, or with grains such as a matte finish pattern or a texture pattern.

As shown in FIG. 6B, on the outer (lower) bottom surface of the cover 3, a protrusion 33 in a cylindrical shape is formed to protrude downward. The protrusion 33 has an outer diameter rather smaller than the inner diameter of the through hole 41 (refer to FIG. 7) of the capturing dish 4 to be described below. The height of the protrusion 33 is equal to that of the through hole 41.

As shown in FIGS. 6A and 6B, the capturing dish 4 includes the first dish half 4a and the second dish half 4b.
The first dish half 4a and the second dish half 4b may also be each referred to as a "divided body".
The first dish half 4a and the second dish half 4b are each semicircular as viewed from above. Combination of a pair of the semicircles makes the capturing dish 4 forms a circular shape as viewed from above.
Semicircular cylindrical recessed portions 41a, 41b are disposed in respective central parts of the first dish half 4a and the second dish half 4b so as to form the through hole 41 (refer to FIG. 7) described above when the halves 4a, 4b are joined to form the capturing dish 4.

As shown in FIG. 6A, the upper surfaces of the first dish half 4a and the second dish half 4b form an even surface to be brought in contact with the outer bottom surface of the cover 3 described above.
On lower surfaces of the first dish half 4a and the second dish half 4b, carrier holding ribs 42a, 42b are vertically provided to hold the count analysis carrier 5a and the identification analysis carrier 5b each in a half disk-shaped, as described below.

The outer diameter of the capturing dish 4 ranges between the inner diameter of a lower cylinder portion 62 and the inner diameter of an upper cylinder portion 61 of the housing 6 (including both end values) . Preferably, the outer diameter of the capturing dish 4 is substantially equal to the inner diameter of the upper cylinder portion 61.

The carrier 5 is placed in the air sampler 50 (refer to FIG. 8) as described below, to receive air flow when the air sampler 50 sucks the air, and to capture microorganisms carried in the air flow.

The carrier 5 includes the count analysis carrier 5a and the identification analysis carrier 5b as described above. The count analysis carrier 5a and the identification analysis carrier 5b are held in the carrier holding ribs 42a, 42b, respectively, to be thereby arranged in sections.
The carrier 5 includes the count analysis carrier 5a and the identification analysis carrier 5b is disposed on a side of one surface of the capturing dish 4. The count analysis carrier 5a and the identification analysis carrier 5b are made to be dividable by the first dish half 4a and the second dish half 4b, respectively.
The carrier 5 is made of a material that undergoes a phase transition from gel to sol when the temperature rises from the room temperature. The material of the carrier 5 is preferably such a material that undergoes a phase transition from gel to sol at 30 degrees C or higher. More preferably, the material liquefies at a temperature between 37 degrees C and 40 degrees C. Most preferably, the material is a gelatin, a mixture of gelatin and glycerol, or a copolymer having a ratio of N-acryloylglycinamide to N-methacryloyl-N'-biotinyl propylene diamine of 10:1.

As shown in FIGS. 6A and 6B, the housing 6 has: the upper cylinder portion 61 having the inner diameter substantially the same as the outer diameter of the cover 3 as described above; the lower cylinder portion 62 having the inner diameter smaller than the inner diameter of the upper cylinder portion 61; a conical portion 64 formed in a substantially inverted conical shape with an inner diameter which gradually becomes smaller from the inner diameter of the lower cylinder portion 62; and a filter fitting portion 65 provided on the periphery of an outlet of the discharge opening 64a formed in the lowest portion of the conical portion 64, which are disposed in this order from upward to downward to form an integral unit.

On an inner circumferential surface of the upper cylinder portion 61, three of the first L-shaped grooves 61a into which the third engaging claws 32 of the cover 3 are fitted are formed at positions corresponding to the third engaging claws 32 as described above.

The lower cylinder portion 62 is connected with the upper cylinder portion 61 through a shelf portion 63. On an outer circumferential surface of the lower cylinder portion 62, the first engaging claws 62a are formed to be engaged with the engaging ring 102b (refer to FIG. 2) of the microorganism counting apparatus 10 described above. The first engaging claws 62a protrude outward in the radial direction of the lower cylinder portion 62, and are disposed in a constant spacing with each other on the circumferential surface of the lower cylinder portion 62. According to the embodiment, the number of the first engaging claws 62a is four.

The conical portion 64 having the inner diameter becoming smaller downward enables the contents to easily flow down toward the lowest portion, that is, the discharge opening 64a (refer to FIG. 6B).

The filter fitting portion 65 forms an integral unit with: a filter housing portion 65a (refer to FIG. 6B) forming a thin disk-shaped space, the shape of which matches that of the filter 7 which is disposed to close the outlet of the discharge opening 64a (refer to FIG. 6B) ; and a ring supporting portion 65b having a cylindrical shape and supporting the filter-securing ring 8.

Second L-shaped grooves 65c are formed on the inner circumferential surface of the ring supporting portion 65b, and fourth engaging claws 82a formed on the filter-securing ring 8 described below are fitted into respective second L-shaped grooves 65c. The number of the second L-shaped grooves 65c is four, and the second L-shaped grooves 65c are formed to be disposed in a constant spacing with each other on the circumferential surface of the ring supporting portion 65b.

The filter 7 according to the embodiment is a membrane filter. As described above, the filter 7 closes the outlet of the discharge opening 64a (refer to FIG. 6B) . In other words, the filter 7 is disposed on the outside of the discharge opening 64a. The filter 7 includes a hydrophilic filter 7a and a hydrophobic filter 7b, which are arranged in this order viewed from the discharge opening 64a.

The hydrophilic filter 7a and the hydrophobic filter 7b may be selected from commercially available products. Examples of the hydrophilic filter 7a include MF-Millipore (manufactured by Nihon Millipore K.K.), Durapore (Nihon Millipore K.K.), and Isopore (Nihon Millipore K.K.).
Examples of the hydrophobic filter 7b include Mitex (Nihon Millipore K.K.) and Polypropylene Prefilter (Nihon Millipore K.K.) .
Note that the filter 7 used in the embodiment should have an outer diameter larger than the inner diameter of the discharge opening 64a (refer to FIG. 6B).

As shown in FIGS. 6A and 6B, the filter-securing ring 8 fixes the filter 7 to the housing 6 (i.e., the conical portion 64). The filter-securing ring 8 has a through hole 81 at a position where the through hole 81 communicates with the discharge opening 64a of the conical portion 64 through the filter 7.

The filter-securing ring 8 includes a ring body 82 having a shape substantially same as the inner diameter of the ring supporting portion 65b of the filter fitting portion 65 described above, and a flange portion 83 formed on the lower side of the ring body 82 and having a diameter larger than the outer diameter of the ring body 82.

As shown in FIG. 6A, the filter-securing ring 8 further includes: a fitting portion 84 which is deposited on the ring body 82 so that the fitting portion 84 and the ring body 82 form an integral unit, and is fitted into the filter housing portion 65a of the housing 6; and a ring-shaped rib 85 vertically disposed on the periphery of an opening of the through hole 81 of the fitting portion 84. The ring-shaped rib 85 presses the filter 7 on the periphery of the outlet of the discharge opening 64a (refer to FIG. 6B).

On the circumferential surface of the ring body 82, four of the fourth engaging claws 82a (refer to FIG. 6B) are formed to protrude radially outward and are disposed in a constant spacing with each other on the circumferential surface of the ring body 82. The fourth engaging claws 82a are formed at positions corresponding to the respective second L-shaped grooves 65c of the ring supporting portion 65b described above, and are fitted into the respective second L-shaped grooves 65c to detachably engage the filter-securing ring 8 with the housing 6.

As shown in FIG. 7, the device for capturing object 1 as described above is formed as follows: the first dish half 4a and the second dish half 4b are combined together to form the disk-shaped capturing dish 4; the capturing dish 4 is mounted on the shelf portion 63 of the housing 6; the housing 6 is coupled to the cover 3 through the capturing dish 4 using the first L-shaped grooves 61a and the third engaging claws 32; and the through hole 41 of the capturing dish 4 is sealed by the protrusion 33 of the cover 3.
The housing 6 is decoupled from the cover 3 by rotating the housing 6 relative to the cover 3 to disengage the third engaging claws 32 from the first L-shaped grooves 61a.

The filter 7 is disposed in the filter housing portion 65a to close the outlet of the discharge opening 64a of the conical portion 64, and the filter fitting portion 65 is engaged with the filter-securing ring 8 using the second L-shaped grooves 65c and the fourth engaging claws 82a described above. Thereby, the discharge opening 64a of the conical portion 64 communicates with the through hole 81 of the filter-securing ring 8 through the filter 7. As described above, when the filter fitting portion 65 is engaged with the filter-securing ring 8, the filter 7 is pressed by the ring-shaped rib 85 of the filter-securing ring 8, and thereby the filter 7 is disposed on the periphery of the outlet of the discharge opening 64a. Thus, the filter 7 is fixed firmly.

In the device for capturing object 1, as shown in FIG. 7, the protrusion 33 of the cover 3 seals the through hole 41. The outlet of the discharge opening 64a of the conical portion 64 is closed by the filter 7 which separates microorganisms. As a result, the internal space 66 is a space isolated from the external environment (i.e., a closed space) at least for microorganisms. Consequently, the count analysis carrier 5a held on the first dish half 4a and the second dish half 4b is placed in this closed space. The device for capturing object 1 as described above other than the filter 7 may be molded with resin, preferably polypropylene.

### <Method for Using Device for Capturing Object>

A method for using the device for capturing object 1 according to the first embodiment will be described next.
First, a method for capturing microorganisms using the device for capturing object 1 will be described. FIG. 8 referred to next is a perspective view showing the method for capturing microorganisms using the device for capturing object of the present invention. FIG. 9 is a perspective view showing how a carrier is divided in the device for capturing object according to the first embodiment.

As shown in FIG. 8, when microorganisms are captured, the device for capturing object 1 (refer to FIG. 7) is used in such a way that the first dish half 4a holding the count analysis carrier 5a and the second dish half 4b holding the identification analysis carrier 5b are combined together to form the capturing dish 4, and then, the capturing dish 4 is mounted on the cover 3. In other words, the device for capturing object 1 shown in FIG. 7 is turned upside down and is used with the first dish half 4a and the second dish half 4b left on the cover 3 and with the housing 6 and the filter-securing ring 8 removed. The housing 6 is removed from the cover 3 by rotating the housing 6 relative to the cover 3 to disengage the third engaging claws 32 (refer to FIG. 6A) from the first L-shaped grooves 61a (refer to FIG. 6A) as described above after the cover 3 is located in the pedestal 52 of the air sampler 50 as described below.

The device for capturing object 1 is mounted on the pedestal 52 formed of a round shape as viewed from above, which is formed on the upper side of an air sampler body 51 of the air sampler 50. As described above, the pedestal 52 has the cutout portions 53 formed to receive the second engaging claws 31 of the cover 3, and the device for capturing object 1 is thereby located in a center portion of the pedestal 52.

FIG. 8 shows suction openings 54 of the air sampler body 51, a nozzle head 55 of the air sampler 50, and a nozzle 55a disposed in the nozzle head 55. The nozzle 55a is configured by a disk-shaped plate with a plurality of very small nozzle holes formed thereon.

According to the method for capturing microorganisms, the housing 6 and the filter-securing ring 8 which form an integral unit are removed to expose the carrier 5 of the device for capturing object 1 mounted in the pedestal 52, and the nozzle head 55 is placed over the pedestal 52, as shown in FIG. 8.

A fan not shown disposed in the air sampler body 51 is activated, and the air is sucked through the suction openings 54. Then, air flow is injected to the carrier 5 from multiple nozzle holes of the nozzle 55a provided in the nozzle head 55. As a result, microorganisms carried in the air injected to the carrier 5 are captured by the count analysis carrier 5a and the identification analysis carrier 5b. In other words, microorganisms are captured with the carrier 5 directed upward.

As shown in FIG. 8, the protrusion 33 of the cover 3 seals the through hole 41 (refer to FIG. 7) of the capturing dish 4. Thus, the surface of the capturing dish 4 on the side of the carrier 5 is flush with the bottom of the protrusion 33. This reduces disturbance of the received air flow. Consequently, the carrier 5 can capture microorganisms efficiently.
When the air sampler 50 sucks a predetermined amount of the air, the process of capturing microorganisms with the device for capturing object 1 ends.
When the capturing process ends, the housing 6 and the filter-securing ring 8 which form an integral unit are fitted to the cover 3 again, and the device for capturing object 1 returns back to the state shown in FIG. 7.

A method for using the device for capturing object 1 in the microorganism counting apparatus 10 which counts the captured microorganisms will be described.
When the capturing process described above ends, the device for capturing object 1 as shown in FIG. 7 is carried by a user to a place where the microorganism counting apparatus 10 (refer to FIG. 1) is installed.
The housing 6 is removed from the cover 3 by the user.
After the first dish half 4a which holds the count analysis carrier 5a is removed from the cover 3 as shown in FIG. 9, the first dish half 4a is mounted on the mounting unit 102 together with the housing 6 as shown in FIG. 3.
On the other hand, the first cover body 35 is attached to the cover 3 on which the second dish half 4b holding the identification analysis carrier 5b is still placed as shown in FIG. 9, in such a manner that the first cover body 35 covers the identification analysis carrier 5b.
The first cover body 35 has a cylindrical shape with a bottom. Third L-shaped grooves 35a are formed on an inner circumferential surface of an opening of the housing 6. The opening of the housing 6 has an inner diameter substantially same as that of the upper cylinder portion 61 (refer to FIG. 6A) .
The third L-shaped grooves 35a has a structure same as that of the first L-shaped grooves 61a (refer to FIG. 6A) of the housing 6.
The third engaging claws 32 of the cover 3 are fitted into the respective third L-shaped grooves 35a of the first cover body 35 to detachably engage the cover 3 with the first cover body 35.
As a result, the first cover body 35 seals the identification analysis carrier 5b between itself and the cover 3.
The identification analysis carrier 5b is subjected to an identification analysis for identifying types of microorganisms.
Note that the count analysis may also be referred to as a "first detection operation of substances to be detected". The identification analysis may also be referred to as a "second detection operation of substances to be detected".

Next is described a method for using the device for capturing object 1 in the microorganism counting apparatus 10 for counting captured microorganisms.

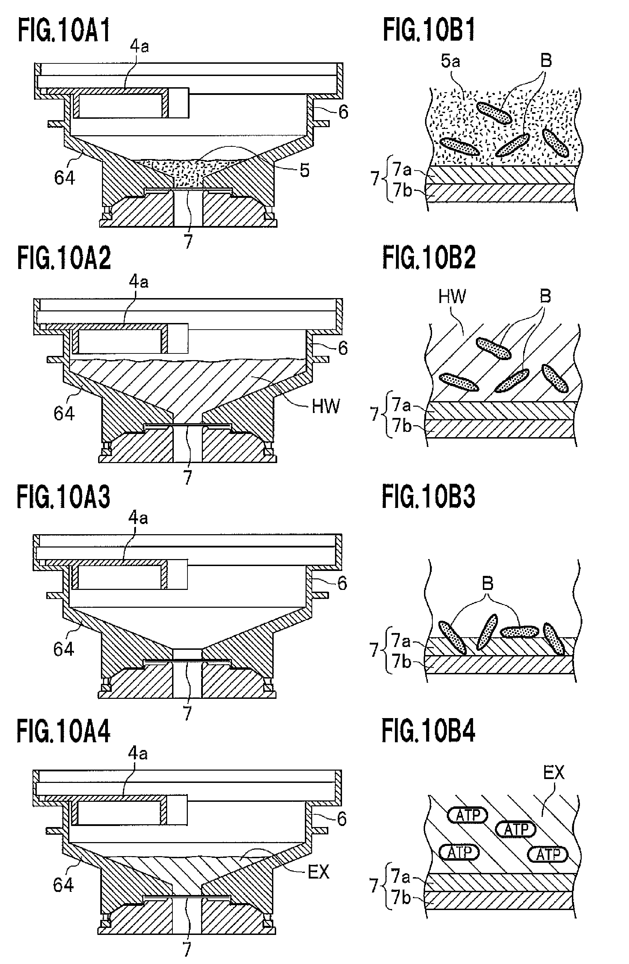

FIGS. 10A1 to 10A4 to be referred next are cross-sectional views of the device for capturing object, showing the method for using the device for capturing object in the microorganism counting apparatus. FIGS. 10B1 to 10B4 are enlarged schematic diagrams showing the vicinity of the filter in the case of FIGS. 10A1 to 10A4.
FIGS. 10B1 to 10B4 show microorganisms B and ATPs . Sizes of actual microorganisms are, however, as small as in the order of micrometer, and sizes of actual ATPs are as small as that of a molecule. Accordingly, FIGS. 10B1 to 10B4 shows no relative sizes of a microorganism and an ATP.

When the temperature of the carrier 5a is raised in Step S201 (refer to FIG. 4) as described above, the count analysis carrier 5a held on the first dish half 4a solates and falls down onto the conical portion 64 of the housing 6 as shown in FIG. 10A1. In this step, the microorganisms B captured with the air sampler 50 (refer to FIG. 8) are retained with the count analysis carrier 5a on the filter 7 as shown in FIG. 10B1.

When hot water HW is injected into the housing 6 in Step S202 (refer to FIG. 4) as described above, the count analysis carrier 5a further solates and is diluted by the hot water. The filter 7 includes the hydrophobic filter 7b on the lower side thereof as shown in FIG. 10B2. Thus, the hot water HW containing the diluted count analysis carrier 5a (refer to FIG. 10A1) is retained in the housing 6. The microorganisms B are retained in the hot water HW on the filter 7. In FIG. 10A2, reference numeral 4a indicates the first dish half 4a, and reference numeral 64 indicates the conical portion 64 (hereinafter the same).

When the contents in the housing 6 are filtered in Step S203 (refer to FIG. 4) as described above, the hot water HW in the housing 6 (refer to FIG. 10A2) is discharged as shown in FIG. 10A3. In this step, the microorganisms B in the hot water HW are separated and held by the filter 7 as shown in FIG. 10B3.

As shown in FIG. 10B3, the filter 7 according to the embodiment has a double layer structure of the hydrophilic filter 7a and the hydrophobic filter 7b. Unlike a filter including only a hydrophilic filter used in conventional ATP methods, the hydrophobic filter 7b enables liquid to be retained on the filter unless the liquid is sucked or pressure-filtered. This enables reaction with reagent, such as ATP extraction, to be performed on the filter 7.

The ATP eliminating reagent is dispensed into the housing 6 in Step S206 (refer to FIG. 4) as described above, and then the ATP extracting reagent is dispensed into the housing 6 in Step S209 (refer to FIG. 4) as described above.
These processes of dispensing the reagents may also be referred to as "a step of injecting a reagent into the housing".

In the housing 6 into which the ATP extracting reagent is injected in Step S209 (refer to FIG. 4) as described above, an ATP extract solution EX is retained as shown in FIG. 10A4. As shown in FIG. 10B4, the ATP extract solution EX contains ATPs, amount of which corresponds to the number of the microorganisms B.

The ATP extract solution EX shown in FIG. 10B4 is dispensed into the luminescence-test tube 107a (refer to FIG. 1) in Step S210 (refer to FIG. 4) as described above. The microorganisms are then counted through Step S211 and Step S212 (refer to FIG. 4) .
If it is confirmed that there are microorganisms in the count analysis carrier 5a, the identification analysis carrier 5b held on the second dish half 4b still left on the cover 3 shown in FIG. 9 is subjected to the identification analysis, to thereby types of the microorganisms.

According to the device for capturing object 1 and the method for using the same as described above, the carrier 5 including the count analysis carrier 5a and the identification analysis carrier 5b is disposed on a side of one surface of the capturing dish 4. Thus, when the capturing dish 4 is placed in the air sampler 50 (refer to FIG. 8) and the carrier 5 receives air flow sucked by the air sampler 50, the count analysis carrier 5a and the identification analysis carrier 5b can capture microorganisms such that the respective numbers of the microorganisms per unit area (exposed area) on the carriers 5a, 5b are substantially the same.

According to the device for capturing object 1 and the method for using the same, the carrier 5 is dividable into the count analysis carrier 5a and the identification analysis carrier 5b, which are disposed individually. Unlike the carrier 5 of an integral type, this eliminates the need of, after capture of microorganisms, cutting the carrier 5 into pieces and weighing each of the pieces.
Thus, according to the device for capturing object 1 and the method for using the same, when the cutting or weighing is performed, substances which become disturbance factors for counting microorganisms can be prevented from being mixed into the carrier 5. As a result, according to the device for capturing object 1 and the method for using the same, when the carrier 5 after capture of microorganisms is subjected to a plurality of analyses such as, for example, a quantitative analysis and a qualitative analysis, those analyses can be performed further accurately.

According to the device for capturing object 1 and the method for using the same as described above, microorganisms are captured with the carrier 5 directed upward, and then the carrier 5 is directed downward to contact the microorganisms with the reagents.
Thus, according to the device for capturing object 1 and the method for using the same, the carrier 5 directed upward facilitates the capturing of the microorganisms. Also, when the reagents are contacted with the microorganisms, that is, the microorganisms are detected, the carrier 5 is directed downward, and thereby the capturing dish 4 serves as a cover of the carrier 5. For example, this prevents the carrier 5 from being contaminated with dust, microbes, or the like falling from above.

Before the device for capturing object 1 is mounted in the air sampler 50, and during the time after the microorganisms are captured using the air sampler 50 and before the captured microorganisms are carried into the microorganism counting apparatus 10, the carrier 5 is placed in the closed space in the housing 6. As a result, the carrier 5 is prevented from being contaminated with substances which are disturbance factors for the counting of the microorganisms, unlike a conventional device for capturing object with an exposed carrier, such as the device disclosed in Japanese Patent Application Laid-Open No. 2009-131186.
Consequently, the device for capturing object 1 and the method for using the same enable more accurate counting of the microorganisms captured at a test site.

According to the device for capturing object 1 and the method for using the same, the housing 6 has the discharge opening 64a through which the contents thereof are discharged, and the discharge opening 64a has the filter for separating and holding microorganisms. Thereby, the microorganisms can be contacted with the reagents R in the housing 6. Consequently, the device for capturing object 1 and the method for using the same dramatically reduce the disturbance factors for the counting of the microorganisms, unlike a conventional device for capturing object (for example, see Patent Document 1 used in such a way that microorganisms are extracted from the device for capturing object and the extracted microorganisms are contacted with reagents for counting.

According to the device for capturing object 1 and the method for using the same, the filter 7 has a double layer structure of the hydrophilic filter 7a and the hydrophobic filter 7b. Thereby, reaction of reagents with recovered microorganisms can be performed on the filter 7, unlike a filter used in the conventional ATP methods which includes only a hydrophilic filter.

According to the device for capturing object 1 and the method for using the same, the cover 3 has the second engaging claws 31 formed on the opposite side of the housing 6 to engage with the air sampler. To expose the carrier 5, the housing 6 in a state of forming an integral unit with the cover 3 as shown in FIG. 5 is grasped by hands, the cover 3 is placed into the air sampler 50 as shown in FIG. 8, and then, the housing 6 is removed from the cover 3. In other words, when the carrier 5 is exposed, contact between the capturing dish 4 holding the carrier 5 and hands and fingers can be prevented. Consequently, the device for capturing object 1 and the method for using the same can surely prevent the carrier 5 from being contaminated with substances as disturbance factors for the counting of the microorganism.

According to the device for capturing object 1 and the method for using the same, after the device for capturing object 1 is mounted on the mounting unit 102, when the cover 3 is removed from the housing 6 by a user, the first dish half 4a is turned upside down relative to the state at the time when placed in the air sampler, and thereby the count analysis carrier 5a faces toward the internal space 66. This can surely prevent the contamination of the count analysis carrier 5a.

The first embodiment of the present invention has been explained as aforementioned. However, the present invention is not limited to the embodiment described above and can be carried out with various modifications.
In the embodiment described above, the capturing dish 4 is configured to include two divided bodies, the first dish half 4a and the second dish half 4b. However, the capturing dish 4 may be configured to include three or more divided bodies.

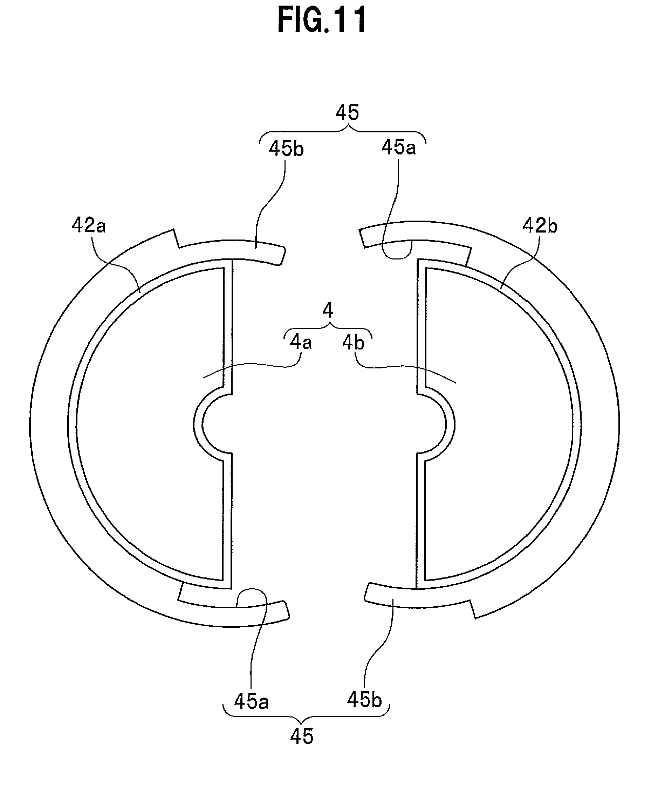

When the first dish half 4a and the second dish half 4b are combined together to form the capturing dish 4, the first dish half 4a and the second dish half 4b may each have an engaging means so as to engage with each other. FIG. 11 to be refereed next is an exploded plan view showing a structure of a capturing dish according to a variation.

As shown in FIG. 11, in the capturing dish 4, the first dish half 4a and the second dish half 4b include engaging means 45 each including an engaging convex portion 45b and an engaging concave portion 45a. The engaging convex portion 45b and the engaging concave portion 45a are fitted in with each other and are disposed at peripheral edges of the respective halves 4a, 4b avoiding positions where respective carrier holding ribs 42a, 42b, are present.
The capturing dish 40 having the engaging means 45 as described above can dispose the first dish half 4a and the second dish half 4b in the housing 6 (refer to FIG. 7) or in the nozzle head 55 (refer to FIG. 8) of the air sampler 50 more stably. Further, the capturing dish 4 having the engaging means 45 can be conveniently carried around because the first dish half 4a and the second dish half 4b can be made into an integral unit.
However, the engaging means 45 is not limited to the integral unit with the first dish half 4a and the second dish half 4b. The engaging means 45 may be configured by a member disposed separately therefrom, such as, for example, a clip.

In the embodiment described above, when a counting analysis of microorganisms is performed using the microorganism counting apparatus 10 (refer to FIG. 1), the second dish half 4b (refer to FIG. 3) holding the identification analysis carrier 5b (refer to FIG. 3) is removed from the housing 6 (refer to FIG. 3), and then, the housing 6 is mounted on the mounting unit 102. However, the second dish half 4b holding the identification analysis carrier 5b may not be removed from the housing 6, and the identification analysis carrier 5b may also be subjected to the counting analysis. In this case, the reagent R or the like is dispensed in the housing 6 via the through hole 41.
It is easily understood that both the count analysis carrier 5a and the identification analysis carrier 5b may be subjected to the counting analysis.

In the embodiment described above, the counting analysis is performed and, if it is confirmed that microorganisms are present in the count analysis carrier 5a, then an identification analysis of the identification analysis carrier 5b is performed. However, the identification analysis may be performed in parallel with the counting analysis.

In the embodiment described above, microorganisms captured by the device for capturing object 1 are counted using the microorganism counting apparatus 10. However, the present invention is applicable to a case in which the reagent R is manually dispensed into the housing without incorporating the device for capturing object 1 into the microorganism counting apparatus 10, and the microorganisms are counted using the ATP method.

The present invention is applicable to spore-forming bacteria such as Bacillus subtilis. In this case, examples of the reagents described above may include a vegetative cell conversion reagent such as amino acid and sugar.

In the embodiment described above, the ATP method is used to count microorganisms. Instead, the microorganisms may be counted based on the fluorescence produced when substances in a living body such as DNA, RNA, and NAD extracted from the microorganisms are irradiated with excitation light.

In the case where the device for capturing object 1 is used to capture and count gram negative bacilli, the counting may be made based on endotoxin contained in the cell membrane of gram negative bacilli. In other words, microbes may be counted based on luminescence intensity resulting from the limulus test on the endotoxin.

The microorganisms may be counted after being recovered from the filter 7 and cultured.

In the embodiment described above, the carrier 5 is configured to be divided into plural portions which are then disposed separately in sections, by dividing the capturing dish 4 into plural pieces. However, the present invention is not limited to the embodiment described above as long as the carrier 5 can be divided into plural portions which are disposed separately in sections. For example, the carrier 5 may be configured as shown next in a second embodiment.

### <Second Embodiment>

Next will be described a device for capturing object according to a second embodiment of the present invention.
Herein, a microorganism counting apparatus equipped with the device for capturing object and a method for counting microorganisms by the microorganism counting apparatus according to this embodiment are the same as those according to the first embodiment. Thus, detailed description thereof is omitted herefrom.

### <Device for Capturing Object>

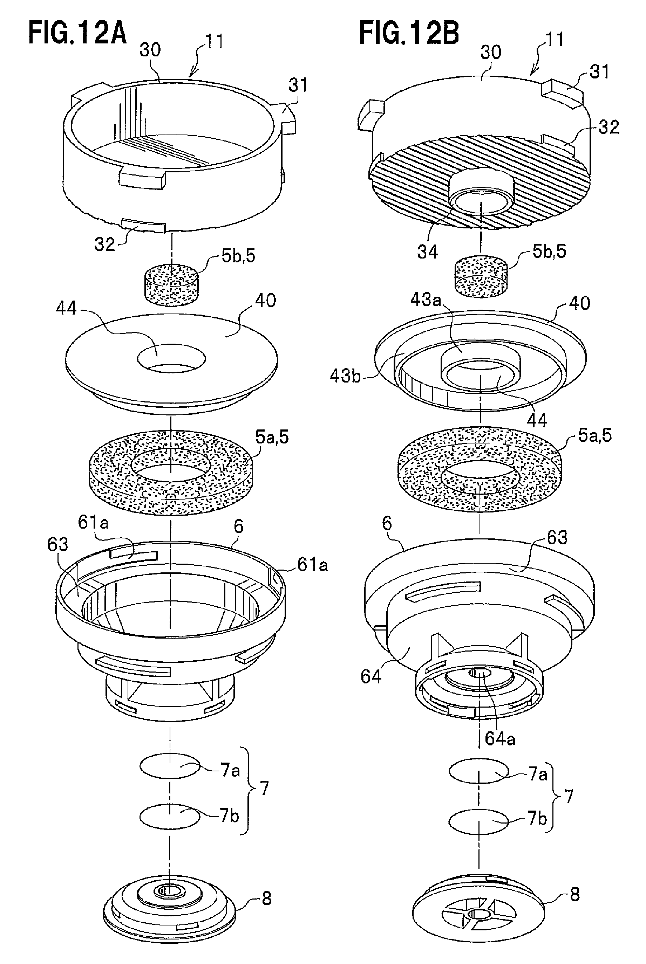

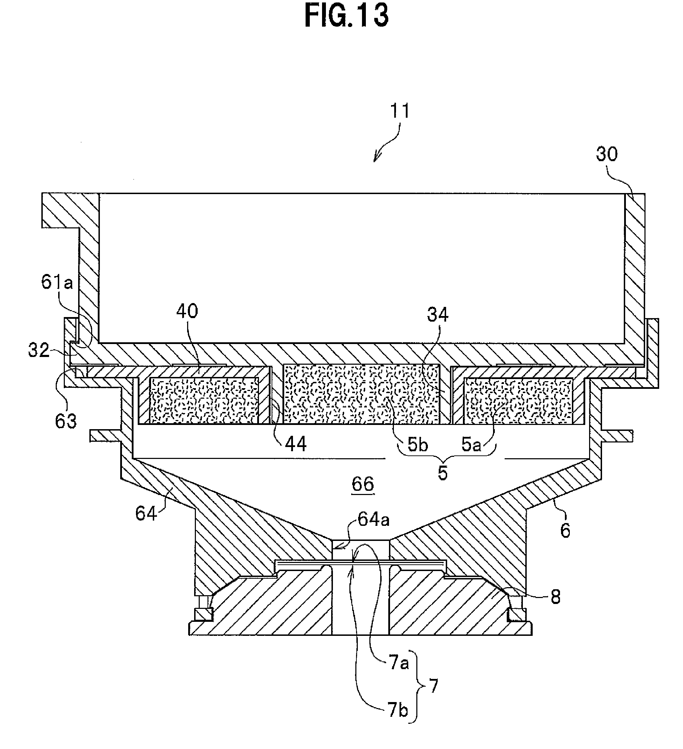

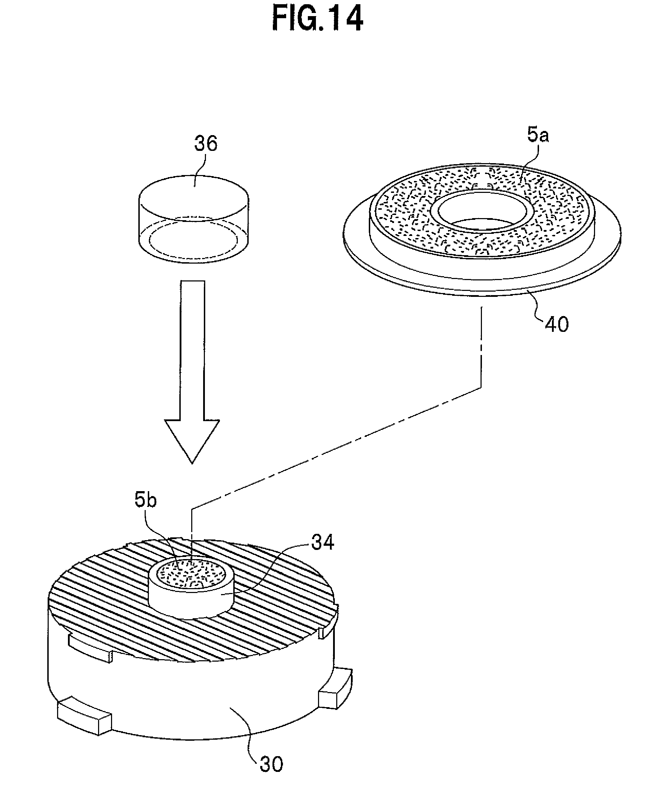

FIG. 12A to be referred to next is an exploded perspective view showing a device for capturing object viewed from obliquely above according to the second embodiment. FIG. 12B is an exploded perspective view showing the device for capturing object viewed from obliquely below according to the second embodiment. FIG. 13 is a cross-sectional view along a line VII-VII in FIG. 5 according to the second embodiment. FIG. 14 is a perspective view showing how a carrier is divided in the device for capturing object according to the second embodiment.
In the second embodiment, the same reference numerals are given to the components similar to those in the first embodiment, and detailed description thereof is omitted herefrom.
As shown in FIG. 12A and FIG. 12B, a device for capturing object 11 also includes the housing 6, the filter 7, and the filter-securing ring 8 which are the same as those of the device for capturing object 1 (refer to FIG. 6A and FIG. 6B) according to the first embodiment, except that the device for capturing object 11 further includes a capturing dish 40 having the count analysis carrier 5a and a cover 30 having the identification analysis carrier 5b. The following description thus focuses on the cover 30 and the capturing dish 40.

On an outer surface of the cover 30, a ring-like carrier holding rib 34 is vertically provided, instead of the protrusion 33 (refer to FIG. 6A and FIG. 6B) formed on the outer surface of the bottom of the cover 3 according to the first embodiment. The outer diameter of the carrier holding rib 34 is slightly smaller than the inner diameter of the through hole 44 of the capturing dish 40 to be described next. The height of the carrier holding rib 34 is equal to that of the through hole 44.
In the cover 30 shown in FIG. 12A and FIG. 12B, reference numeral 31 indicates a second engaging claw which engages with the cutout portion 53 of the air sampler 50 shown in FIG. 8. Reference numeral 32 indicates a third engaging claw which is fitted into the first L-shaped groove 61a of the housing 6.

As shown in FIG. 12A and FIG. 12B, the capturing dish 40 is formed of a disk. In the central part of the capturing dish 40, the through hole 44 is formed which penetrates the capturing dish 40 from a lower surface side to an upper surface side thereof. The lower surface side of the capturing dish 40 may also be referred to as "one surface side". The upper surface side of the capturing dish 40 may also be referred to as "the other surface side".

The upper surface of the capturing dish 40 is flat as shown in FIG. 12A so as to allow a contact with the outer surface of the bottom of the cover 30 described above.
On the lower surface of the capturing dish 40, the inner and outer double ring-shaped carrier holding ribs 43a, 43b are vertically provided surrounding the through hole 44.

The carrier 5 in this embodiment includes, as described above, the identification analysis carrier 5b which is accommodated in the carrier holding rib 34 of the cover 30, and the count analysis carrier 5a which is accommodated between the inner and outer double ring-shaped carrier holding ribs 43a, 43b of the capturing dish 40. The identification analysis carrier 5b may also be referred to as a "second divided portion of the carrier".
The identification analysis carrier 5b is formed of a column in conformity to a shape of the space in the carrier holding rib 34. The count analysis carrier 5a is formed of a ring in conformity to a shape of the space between the carrier holding ribs 43a, 43b.
In FIG. 12A and FIG. 12B, reference numeral 7 indicates the filter which includes the hydrophilic filter 7a and the hydrophobic filter 7b. Reference numeral 8 indicates the filter-securing ring; 63, the shelf portion; and 64a, the discharge opening of the conical portion.

As shown in FIG. 13, in the device for capturing object 11 as described above, the capturing dish 40 is placed on the shelf portion 63 described above of the housing 6, and the housing 6 and the cover 30 are engaged with each other via the capturing dish 40 by means of the first L-shaped groove 61a and the third engaging claw 32. At this time, the through hole 44 of the capturing dish 40 is sealed by the carrier holding rib 34 of the cover 30 and the identification analysis carrier 5b accommodated therein.
In other words, because the carrier holding rib 34 of the cover 30 is fitted into the through hole 44 of the capturing dish 40, the identification analysis carrier 5b accommodated in the carrier holding rib 34 faces a lower surface side (one surface side) of the capturing dish 40.

Similarly to the device for capturing object 1 (refer to FIG. 7) according to the first embodiment, in the device for capturing object 11 shown in FIG. 13, an outlet of the discharge opening 64a of the conical portion 64 is covered by the filter 7 (the hydrophilic filter 7a and the hydrophobic filter 7b) for separating microorganisms. As a result, the internal space 66 is defined as a space separated from external environment (a closed space) in terms of at least microorganisms. Then, the count analysis carrier 5a held by the capturing dish 40 and the identification analysis carrier 5b held by the cover 30 are disposed in the closed space.
In FIG. 13, reference numeral 8 indicates the filter-securing ring.

### <Method for Using the Device for Capturing Object>

Next is described a method for capturing microorganisms by the device for capturing object 11.
When airborne microorganisms are captured using the device for capturing object 11, as shown in FIG. 13, an assembled unit which includes: the capturing dish 40 installed to the cover 30; the count analysis carrier 5a; and the identification analysis carrier 5b, is placed on the pedestal 52 of the air sampler 50. The configuration described above can be compared to that in the first embodiment with reference to FIG. 8, in which the first dish half 4a and the count analysis carrier 5a as well as the second dish half 4b and the identification analysis carrier 5a is placed on the cover 3.
The count analysis carrier 5a and the identification analysis carrier 5b capture microorganisms in a similar way to that in the first embodiment.

In this case, in FIG. 13, the carrier holding rib 34 of the cover 30 and the identification analysis carrier 5b seals the through hole 44 of the capturing dish 40, in a similar way to that, in FIG. 8, the protrusion 33 of the cover 3 seals the through hole 41 (refer to FIG. 7) of the capturing dish 4. The sealing of the through hole 44 makes the count analysis carrier 5a flush with the identification analysis carrier 5b, to thereby suppress turbulence of received air flow. This results in an efficient capture of microorganisms by the count analysis carrier 5a and the identification analysis carrier 5b.
After the capturing step described above, the device for capturing object 11 returns to a state shown in FIG. 13 and is then moved by a user to a place where the microorganism counting apparatus 10 is installed (refer to FIG. 2).
The user then removes the housing 6 from the cover 30.
As shown in FIG. 14, the capturing dish 40 holding the count analysis carrier 5a is removed from the cover 30. Then, if it is assumed that FIG. 3 in the first embodiment can also be used for explanation herein, similarly to the housing 6 and the first dish half 4a, the capturing dish 40 is disposed on the mounting unit 102 together with the housing 6. The count analysis carrier 5a is subjected to the counting analysis of microorganisms, similarly to the embodiment described above.
On the other hand, as shown in FIG. 14, a second cover body 36 is fitted onto the carrier holding rib 34 holding the identification analysis carrier 5b such that the second cover body 36 covers the identification analysis carrier 5b.
The second cover body 36 has a cylindrical shape with a bottom and has an inner diameter adapted to fit in with the carrier holding rib 34. When it is confirmed that there are microorganisms in the microorganisms count analysis, the identification analysis carrier 5b is subjected to an identification analysis for identifying types of the microorganisms.
Herein, the first cover body 35 (refer to FIG. 9) may be used instead of or together with the second cover body 36.

The device for capturing object 11 and the method for using the same as described above can have advantageous effects same as those of the device for capturing object 1 and the method for using the same according to the first embodiment and can additionally have the following advantageous effects.
According to the device for capturing object 11 and the method for using the same, the capturing dish 40 is disposed in the housing 6 or in the nozzle head 55 of the air sampler 50 in a state where the carrier holding rib 34 of the cover 30 is fitted in the through hole 44 of the capturing dish 40. As a result, the capturing dish 40 can be disposed more stably in the housing 6 or in the nozzle head 55.
According to the device for capturing object 11 and the method for using the same, when the microorganisms are brought in contact with the reagent R using the microorganism counting apparatus 10, the reagent R is dispensed in the housing 6 via the through hole 44 of the capturing dish 40. This is advantageous in that a communication between the inside and outside of the housing 6 is kept as low as the size of the through hole 44. Thus, the inside of the housing 6 is prevented from being contaminated with substances as disturbance factors for the counting of microorganisms.

The second embodiment of the present invention has been explained as aforementioned. The present invention is, however, not limited to the embodiment and can be carried out with various modifications.
In the embodiment, one unit of the capturing dish 40 is provided. However, the capturing dish 40 may be divided into plural pieces, similarly to the capturing dish 4 in the first embodiment.

- 1: device for capturing object
- 3: cover
- 4: capturing dish
- 4a: first dish half
- 4b: second dish half
- 5: carrier
- 5a: count analysis carrier
- 5b: identification analysis carrier
- 6: housing
- 7: filter
- 7a: hydrophilic filter
- 7b: hydrophobic filter
- 8: filter-securing ring
- 11: device for capturing object
- 30: cover
- 33: protrusion
- 34: carrier holding rib
- 40: capturing dish
- 41: through hole
- 42a: carrier holding rib
- 42b: carrier holding rib
- 44: through hole
- 45: engaging means
- 45a: engaging concave portion
- 45b: engaging convex portion
- 64: conical portion
- 64a: discharge opening
- 65: filter fitting portion
- 65a: filter housing portion
- 65b: ring supporting portion
- 66: internal space

## Claims

1. A device for capturing object comprising a carrier which captures substances to be detected and is made to be dividable into plural portions, is placed with the plural dividable portions arranged separately in sections.

2. The device for capturing object according to claim 1, further comprising a capturing dish that holds the carrier,
wherein the capturing dish includes a plurality of divided bodies combined together, and
wherein the carrier is held by the divided bodies.

3. The device for capturing object according to claim 2, further comprising a housing that houses the capturing dish such that the housing covers the carrier,
wherein the capturing dish has a through hole which runs through between one surface side and the other surface side of the capturing dish, and
wherein the through hole of the capturing dish communicates between a space formed between the one surface side and the housing, and an outside.

4. The device for capturing object according to claim 3,
wherein the housing has a discharge opening which discharges contents in the space to the outside of the housing, and a filter is disposed which separates the substances to be detected, at an outer side of the discharge opening.

5. The device for capturing object according to claim 1, further comprising a capturing dish that holds the carrier,
wherein the capturing dish has a through hole which runs through between one surface side and the other surface side of the capturing dish,
wherein the capturing dish holds a first divided portion of the carrier on the one surface side, and
wherein a second divided portion of the carrier is fitted into the through hole of the capturing dish such that the second divided portion faces the one surface side.

6. The device for capturing object according to claim 5, further comprising a housing that houses the capturing dish such that the housing covers the first divided portion of the carrier and the second divided portion of the carrier,
wherein the through hole of the capturing dish which appears when the second divided portion is removed, communicates between a space formed between the one surface side and the housing, and an outside.

7. The device for capturing object according to claim 6,
wherein the housing has a discharge opening which discharges contents in the space to the outside of the housing, and a filter is disposed which separates the substances to be detected, at an outer side of the discharge opening.

8. A method for using a device for capturing object in which a carrier which captures substances to be detected and is made to be dividable into plural portions, is placed with the plural dividable portions arranged separately in sections, the method comprising the steps of:
capturing the substances to be detected by the carrier;
dividing the carrier according to the sections; and
performing a first detection operation of the substances to be detected in which a part of the divided carrier is subjected to the detection operation and performing a second detection operation of the substances to be detected in which the remaining part of the divided carrier is subjected to the detection operation.
